# EUROPÄISCHE PATENTSCHRIFT

(11) **EP 2 637 714 B1**
(45) Veröffentlichungstag und Bekanntmachung des Hinweises auf die Patenterteilung: **31.05.2017**
(21) Anmeldenummer: 11779428.9
(22) Anmeldetag: 08.11.2011
(51) Int. Cl.: F16M 13/02, B25B 5/04, B25B 5/06, A61M 5/14, B25B 5/16

(54) **MANUELL ZU ÖFFNENDER KLEMMHALTER MIT SENSOR**
CLAMPING HOLDER TO BE OPENED MANUALLY AND HAVING A SENSOR
PINCE À OUVERTURE MANUELLE ÉQUIPÉE D'UN CAPTEUR

(30) Priorität: 08.11.2010 DE 102010043574
(43) Veröffentlichungstag der Anmeldung: 18.09.2013
(73) Patentinhaber: Fresenius Medical Care Deutschland GmbH, 61352 Bad Homburg (DE)
(72) Erfinder: BEISSER, Nicolas, 63452 Hanau (DE)
(74) Vertreter: Nordmeyer, Philipp Werner
(86) Internationale Anmeldenummer: PCT/EP2011/069633
(87) Internationale Veröffentlichungsnummer: WO 2012/062744

(56) Entgegenhaltungen:
- EP-A2- 2 060 364
- US-A- 4 378 014
- US-A- 5 567 120
- US-A- 6 112 622
- US-A1- 2012 146 275

## Beschreibung

### Technisches Gebiet

Die Erfindung bezieht sich auf einen manuell zu öffnenden Klemmhalter und insbesondere auf einen manuell zu öffnenden Klemmhalter für die lösbare Befestigung eines Einmalartikels an einer medizinischen Behandlungsvorrichtung.

### Stand der Technik

Medizinische Behandlungsvorrichtungen weisen oft Halterungen für die Befestigung eines medizinischen Einmalartikels an der medizinischen Behandlungsvorrichtung während einer Behandlung auf. Medizinische Behandlungsvorrichtungen nach der vorliegenden Erfindung sind insbesondere Blutbehandlungsvorrichtungen. Blutbehandlungsvorrichtungen können beispielsweise Geräte zur Adsorptionstherapie, Dialysegeräte, Infusionsgeräte oder Geräte zur Plasmapherese sein. Wesentlich ist, dass zur Vorbereitung der Behandlung eines Patienten die medizinische Behandlungsvorrichtung mit einem medizinischen Einmalartikel ausgerüstet wird, der ordnungsgemäß an der medizinischen Behandlungsvorrichtung befestigt wird und nach der Behandlung wieder verworfen wird. Ein solcher Einmalartikel kann beispielsweise ein Adsorber oder ein Dialysefilter sein. Ein weiteres Beispiel ist eine Infusionsflasche, die ebenfalls mittels einer Halterung an einer Infusionsmaschine befestigt wird. Nach der Verabreichung der Infusionsflüssigkeit wird die leere Infusionsflasche dann wieder aus der Halterung entfernt und anschließend entsorgt.

Ein Unterscheidungsmerkmal für die medizinischen Einmalartikel kann unter Anderen deren Dicke sein.

Als Halterungen für die Befestigung eines medizinischen Einmalartikels an einer medizinischen Behandlungsvorrichtung sind im Stand der Technik Federklemmen mit zumindest zwei Klemmbacken weit verbreitet.

Voraussetzung für eine fehlerfreie Behandlung eines Patienten ist in allen diesen Fällen, dass vor Beginn der Behandlung der medizinische Einmalartikel mittels der Halterung an der medizinischen Behandlungsvorrichtung befestigt und somit für die Behandlung bereitgestellt wurde.

Es kann vorkommen, dass versehentlich ein falscher medizinischer Einmalartikel in der Halterung der medizinischen Behandlungsvorrichtung befestigt wird, wodurch beispielsweise einem Patienten eine falsche Infusionsflüssigkeit verabreicht oder ein falscher, für die gewählte Behandlung ungeeigneter Dialysefilter verwendet werden könnte, was schwerwiegende negative Folgen für die Gesundheit des Patienten haben kann.

In der medizinischen Behandlungspraxis, insbesondere bei der Adsorbtionstherapie, die im Folgenden beispielhaft angeführt ist und bei der Endotoxine aus dem Patientenblut durch einen Adsorber entfernt werden, kann es vorkommen, dass der Adsorber, nicht in der dafür vorgesehenen Halterung eingelegt ist, oder dort nicht während der gesamten Behandlungsdauer verbleibt. Das Bedienpersonal übergeht manchmal aus Unachtsamkeit oder Bequemlichkeit den Schritt, den Adsorber in der dafür vorgesehenen Halterung zu befestigen. Vielmehr hängt der Adsorber in diesem Fall an den Blutschläuchen, die ihn mit dem extrakorporalen Blutkreislauf des Patienten verbinden. Um eine effiziente und für den Patienten gefahrlose Behandlung zu gewährleisten, ist es aber von großer Bedeutung, dass der Adsorber während der Behandlung in der dafür vorgesehenen Halterung befestigt ist. Es kann nämlich vorkommen, dass die vorgesehene Blutflussrichtung bei der Adsorbertherapie durch die fehlerhafte Bedienung nicht eingehalten wird, oder, dass durch das Gewicht des Adsorbers seine Verbindungen zu den Blutschläuchen abreißen und Blut austritt. Beides bedeutet eine große gesundheitliche Gefahr für den zu behandelnden Patienten.

Es ist daher eine Aufgabe der vorliegenden Erfindung, eine kostengünstige und einfach zu handhabende Vorrichtung sowie ein Verfahren zur Verfügung zu stellen, die das Bedienpersonal einer medizinischen Behandlungsvorrichtung bei der rechtzeitigen und korrekten Bestückung der medizinischen Behandlungsvorrichtung mit medizinischen Einmalartikeln unterstützen, um somit das Potenzial von möglichen Fehlern während einer Behandlung zu reduzieren.

### Zusammenfassung der Erfindung

Die Aufgabe wird von einem Klemmhalter und einem Verfahren gemäß den Gegenständen der unabhängigen Ansprüche gelöst. Vorteilhafte und/oder alternative Weiterbildungen der Erfindung sind den abhängigen Ansprüchen zu entnehmen.

Die US 4,378,014 betrifft eine Vorrichtung und ein Verfahren zum Verabreichen intravenöser Fluide.

Die EP 2 060 364 A2 betrifft eine mechanisch wirkende Spannvorrichtung für Bauteile.

Die US 5,567,120 betrifft eine elektronische Infusionsvorrichtung und einen Rollerklemmehalter für diese.

Die US 2012/0146275 A1 betrifft eine Klemmvorrichtung.

Die US 6 112 622 A1 betrifft eine fluidbetriebene Ratsche zum Fixieren oder Lockern von Befestigungsmitteln.

Ein erfindungsgemäßer Klemmhalter und ein erfindungsgemäßes System kann in jeder medizinischen Behandlungsvorrichtung eingesetzt werden, bei der ein Einmalartikel in einer Klemmhalterung befestigt wird. Beispiele für solche medizinischen Behandlungsvorrichtungen sind Blutbehandlungsgeräte und Infusionsmaschinen. Bei den Blutbehandlungsgeräten sind insbesondere Geräte zur Adsorbertherapie, sowie Dialysegeräte zu nennen. Dabei fallen unter die hier verwendete Definition des Begriffs Dialysegerät alle Geräte und Maschinen zur Durchführung einer Dialyse wie beispielsweise Hämodialysemaschinen oder Maschinen zur automatischen Peritonealdiaylse.

Mit einem erfindungsgemäßen Klemmhalter kann jedweder Einmalartikel, der zur Verwendung mit einer medizinischen Behandlungsvorrichtung vorgesehen ist, an der medizinischen Behandlungsvorrichtung lösbar befestigt werden. Beispiele für Einmalartikel sind: Adsorber, Dialysefilter und Infusionsflaschen.

Ein erfindungsgemäßer Klemmhalter weist zusammenwirkende Klemmbacken auf, die beim Halten eines Einmalartikels zusammenwirken, indem sie den Einmalartikel zwischen sich einklemmen. Mindestens eine der Klemmbacken lässt sich zwischen einer geschlossenen Stellung der Klemmbacken und einer vollständig geöffneten Stellung der Klemmbacken bewegen. In der geschlossenen Stellung der Klemmbacken lassen sich die Klemmbacken nicht weiter zusammendrücken. Die mindestens eine bewegliche Klemmbacke wird durch die Wirkung eines Schließelements in die geschlossene Stellung der Klemmbacken bewegt. Von der geschlossenen Stellung der Klemmbacken lässt sich die mindestens eine bewegliche Klemmbacke gegen die Wirkung des Schließelements bis in die vollständig geöffnete Stellung der Klemmbacken manuell bewegen. In der vollständig geöffneten Stellung der Klemmbacken lassen sich die Klemmbacken nicht weiter auseinander spreizen.

Ein erfindungsgemäßer Klemmhalter weist ferner einen Sensor zur Erfassung der Stellung der Klemmbacken auf.

In einer bevorzugten Ausführungsform eines erfindungsgemäßen Klemmhalters erfasst der Sensor, ob sich die Klemmbacken in der geschlossenen Stellung der Klemmbacken oder nicht in der geschlossenen Stellung der Klemmbacken befinden. Mit dieser Ausführungsform eines erfindungsgemäßen Klemmhalters ist daher sicher feststellbar, ob ein Einmalartikel in dem Klemmhalter eingespannt ist oder nicht. Bei nicht eingespanntem Einmalartikel befinden sich alle Klemmbacken in der geschlossenen Stellung der Klemmbacken, während sich die mindestens eine bewegliche Klemmbacke nicht in der geschlossenen Stellung der Klemmbacken befindet, wenn ein Einmalartikel zwischen den Klemmbacken eingespannt ist.

In einer weiteren bevorzugten Ausführungsform eines erfindungsgemäßen Klemmhalters erfasst der Sensor die Stellung mindestens eines Klemmbackens in einem Bereich zwischen der geschlossenen Stellung der Klemmbacken und einer vollständig geöffneten Stellung der Klemmbacken. Diese Ausführungsform eines erfindungsgemäßen Klemmhalters ermöglicht neben der bloßen Erfassung, ob ein Einmalartikel zwischen den Klemmbacken eingespannt ist oder nicht, auch die Erfassung einer Abmessung des Einmalartikels wie beispielsweise der Umfang oder der Durchmesser des Einmalartikels. Durch die Erfassung einer Abmessung des Einmalartikels können dann beispielsweise in einem erfindungsgemäßen System verschiedene Einmalartikel voneinander unterschieden oder der Füllzustand eines Einmalartikels bestimmt werden.

Ein erfindungsgemäßes System weist neben einem erfindungsgemäßen Klemmhalter ferner eine Auswerteeinheit zur Auswertung der vom Sensor erfassten Stellung der Klemmbacken sowie zur Feststellung einer Abweichung der erfassten Stellung der Klemmbacken von einer vorgegebenen Stellung der Klemmbacken auf.

Die Auswerteeinheit führt für die Feststellung einer Abweichung vorzugsweise einen Vergleich der Daten für die vom Sensor erfasste Stellung der Klemmbacken mit Daten für eine vorgegebene Stellung der Klemmbacken durch.

Eine festgestellte Abweichung der erfassten Stellung der Klemmbacken von einer vorgegebenen Stellung der Klemmbacken führt dann zu der gerechtfertigten Annahme, dass die medizinische Behandlungsvorrichtung mit einem falschen Einmalartikel bestückt wurde.

Auf eine anhand des durchgeführten Vergleichs festgestellte Abweichung der erfassten Stellung der Klemmbacken von einer vorgegebenen Stellung der Klemmbacken wird dann vorzugsweise mit Mitteln für eine optische, akustische und/oder haptische Signalisierung hingewiesen.

Neben der Feststellung einer Abweichung können in der Auswerteeinheit die Daten für die vom Sensor erfasste Stellung der Klemmbacken auch für eine Bestimmung des Füllzustandes eines Einmalartikels mit nachgiebiger Wandung verwendet werden. Hierbei werden die Daten für die vom Sensor erfasste Stellung der Klemmbacken verwendet um den Umfang und somit das Volumen einer Flüssigkeit in dem Einmalartikel zu bestimmen.

Ein erfindungsgemäßes Verfahren zur Bestückung einer medizinischen Behandlungsvorrichtung mit einem Einmalartikel zur Verwendung mit der medizinischen Behandlungsvorrichtung, wobei die medizinische Behandlungsvorrichtung einen manuell zu öffnenden Klemmhalter aufweist, der zusammenwirkende Klemmbacken, von denen mindestens eine Klemmbacke gegen die Wirkung eines Schließelements von einer geschlossenen Stellung der Klemmbacken in eine geöffnete Stellung der Klemmbacken manuell zu bewegen ist, und einen Sensor zur Erfassung der Stellung der Klemmbacken umfasst, weist die folgenden Schritte auf:
manuelles Aufhalten der Klemmbacken des Klemmhalters,
Einführen des Einmalartikels zwischen die Klemmbacken,
Loslassen der Klemmbacken,
Schließen der Klemmbacken durch die Wirkung des Schließelements bis zum Kontakt der Klemmbacken mit dem Einmalartikel,
Erfassen der Stellung der Klemmbacken um den Einmalartikel mittels des Sensors,
Übermitteln vom Sensor erfasster Daten bezüglich der Stellung der Klemmbacken an eine Auswerteeinheit,
Vergleichen in der Auswerteeinheit der Daten der erfassten Stellung der Klemmbacken mit Daten für eine vorgegebene Stellung der Klemmbacken und
Signalisieren einer Warnung beim Vorliegen einer Abweichung der Daten der erfassten Stellung der Klemmbacken von den Daten der vorgegebenen Stellung der Klemmbacken.

Ferner weist ein erfindungsgemäßes Verfahren vorzugsweise den Schritt des Blockierens und/oder Unterbrechens der Ausführung von Funktionen auf der medizinischen Behandlungsvorrichtung beim Vorliegen einer Abweichung der Daten der erfassten Stellung der Klemmbacken von den Daten der vorgegebenen Stellung der Klemmbacken auf.

### Kurze Beschreibung der Zeichnungen

Anhand der Zeichnungen wird die Erfindung nachstehend eingehend erläutert.

Es zeigt:
- Fig. 1: eine perspektivische Ansicht einer bevorzugten Ausführungsform der Erfindung,
- Fig. 2: eine Seitenansicht der in Fig. 1 dargestellten Ausführungsform der Erfindung und
- Fig. 3: eine Explosionsdarstellung der in Fig. 1 dargestellten Ausführungsform der Erfindung.

### Beschreibung der Ausführungsformen

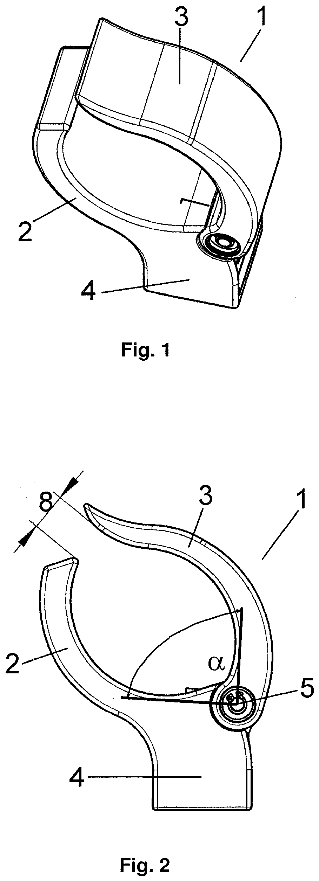

Die perspektivische Darstellung gemäß Fig. 1 zeigt eine bevorzugte Ausführungsform eines erfindungsgemäßen Klemmhalters 1. Der Klemmhalter 1 weist eine erste Klemmbacke 2 und eine zweite Klemmbacke 3 auf. Während die dargestellte Ausführungsform eines erfindungsgemäßen Klemmhalters 1 zwei Klemmbacken 2, 3 aufweist, können weitere bevorzugte Ausführungsformen eines erfindungsgemäßen Klemmhalters auch mehr als zwei Klemmbacken aufweisen. In der in den Figuren dargestellten Ausführungsform eines erfindungsgemäßen Klemmhalters ist die erste Klemmbacke 2 fest mit einem Basisteil 4 des Klemmhalters 1 verbunden und die zweite Klemmbacke 3 ist relativ zu dem Basisteil 4 um eine Drehachse 5 zwischen einer geschlossenen Stellung der Klemmbacken und einer vollständig geöffneten Stellung der Klemmbacken beweglich. Während in der dargestellten Ausführungsform eines erfindungsgemäßen Klemmhalters 1 lediglich eine der Klemmbacken beweglich ist, können in weiteren bevorzugten Ausführungsformen eines erfindungsgemäßen Klemmhaltes mehr als eine Klemmbacke oder sogar alle Klemmbacken beweglich sein.

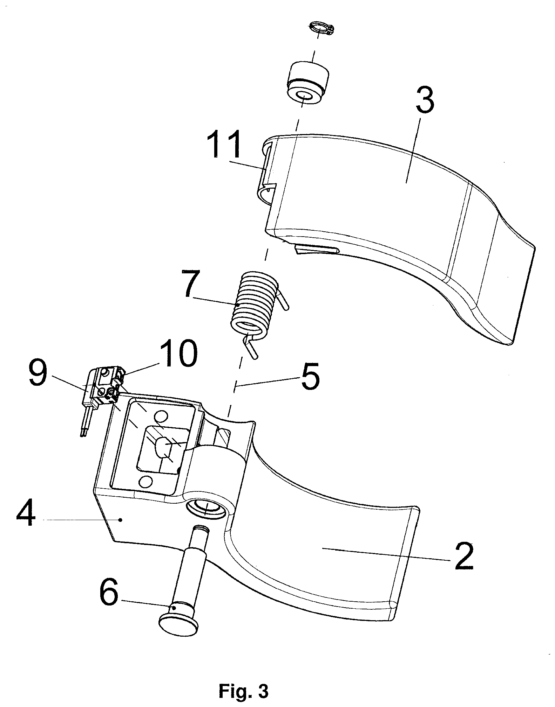

Wie am besten in der Darstellung gemäß Fig. 3 zu sehen ist, wird die zweite Klemmbacke 3 mittels eines Bolzens 6 an dem Basisteil 4 drehbar angeordnet. Die bewegliche Klemmbacke 3 wird von einem Schließelement in die geschlossene Stellung der Klemmbacken bewegt. Hierzu weist das Schließelement eine Schraubendrehfeder 7 auf. Entgegen dem von der Schraubendrehfeder 7 aufgebrachten Drehmoment und somit entgegen der Wirkung des Schließelements ist die bewegliche Klemmbacke 3 durch Aufbringen eines größeren, entgegengesetzten Drehmoments in eine geöffnete Stellung der Klemmbacken manuell zu bewegen. Dies ist leicht zu erreichen, indem eine Bedienperson in jede Hand jeweils eine der beiden Klemmbacken 2, 3 nimmt und dann die bewegliche Klemmbacke 3 um die Drehachse 5 in eine Richtung weg von der ersten Klemmbacke 2 bewegt. Je weiter die beiden Klemmbacken 2, 3 auseinandergespreizt werden, umso größer wird der Winkel α zwischen den Schenkeln der Klemmbacken 2, 3 und umso größer wird der Abstand 8 zwischen den Spitzen der Klemmbacken 2, 3. Der maximale Abstand 8 und größte Winkel α wird erzielt, wenn sich die Klemmbacken 2, 3 in der vollständig geöffneten Stellung der Klemmbacken befinden. In der vollständig geöffneten Stellung der Klemmbacken lassen sich die Klemmbacken 2, 3 nicht weiter auseinanderspreizen.

Anstelle einer Schraubendrehfeder kann das Schließelement des Klemmhalters 1 auch andere aus dem Stand der Technik bekannte Elemente zur Erzielung einer die Klemmbacken in die geschlossene Stellung der Klemmbacken bewegenden Wirkung aufweisen.

Der Klemmhalter 1 wird vorzugsweise mit der Endfläche seines Basisteils 4 an einem Rahmen- oder Gehäuseteil einer medizinischen Behandlungsvorrichtung befestigt. Hierzu sind die aus dem Stand der Technik bekannten Befestigungsmittel wie beispielsweise Schrauben verwendbar. Um einen größeren Abstand zwischen dem Klemmhalter 1 und der medizinischen Behandlungsvorrichtung zu erhalten, kann zwischen den Klemmhalter 1 und das Rahmen- oder Gehäuseteil der medizinischen Behandlungsvorrichtung ein Arm- oder Trägerteil angeordnet werden. Dies ist besonders vorteilhaft für die Anbringung von Einmalartikeln, die zumindest in einem Teilbereich einen großen Umfang aufweisen und daher in einem größeren Abstand von der medizinischen Behandlungsvorrichtung angeordnet werden müssen.

Der Klemmhalter 1 weist ferner einen Sensor 9 zur Erfassung der Stellung der Klemmbacken 2, 3 auf.

Der Sensor eines erfindungsgemäßen Klemmhalters kann ein optischer, magnetischer oder elektromechanischer Sensor sein. Ein Beispiel für einen optischen Sensor ist eine Lichtschranke, die durch eine Leuchtdiode oder eine Laserdiode und einen Fototransistor realisiert werden kann. Ausführungsmöglichkeiten für eine als optischer Sensor eingesetzte Lichtschranke sind dem Fachmann aus dem Stand der Technik bekannt. Als magnetische Sensoren können Reed Relais und Hall-Sensoren verwendet werden. Taster und Potentiometer sind Beispiele für mögliche elektromechanische Sensoren. Dem Fachmann sind weitere Ausführungsmöglichkeiten für den Sensor eines erfindungsgemäßen Klemmhalters aus dem Stand der Technik bekannt.

Der Sensor eines erfindungsgemäßen Klemmhalters kann ein schaltender Sensor sein, mit dem zwei Zustände unterschieden werden können. Mit einem schaltenden Sensor kann somit beispielsweise erfasst werden, ob sich die Klemmbacken in der geschlossenen Stellung der Klemmbacken befinden und daher kein Einmalartikel in dem Klemmhalter eingespannt ist oder sich die Klemmbacken nicht in der geschlossenen Stellung der Klemmbacken befinden, weil ein Einmalartikel in dem Klemmhalter eingespannt ist. Als schaltende Sensoren werden bevorzugt Mikroschalter verwendet.

Der Sensor eines erfindungsgemäßen Klemmhalters kann aber auch ein nicht-schaltender Sensor sein. Mögliche Beispiele hierfür sind Potentiometer und induktive Näherungssensoren. Mit nichtschaltenden Sensoren können die genauen Positionen der Klemmbacken erfasst werden. Dies erlaubt beispielsweise die Erfassung des Winkels α, der von den Klemmbacken 2, 3 gebildet wird. Aus dem Wert des erfassten Winkels α kann dann beispielsweise auf den Umfang oder Durchmesser eines zwischen den Klemmbacken 2, 3 eingeklemmten Einmalartikels geschlossen werden. Dies wiederum erlaubt, auf die Art des Einmalartikels zu schließen, und somit die Bestückung einer medizinischen Behandlungsvorrichtung mit dem richtigen Einmalartikel zu überwachen.

Ein erfindungsgemäßer Klemmhalter kann auch mehr als einen Sensor zur Erfassung der Stellung der Klemmbacken aufweisen. So kann die Stellung jeder Klemmbacke von einem eigenen Sensor erfasst werden. Möglich ist auch, dass einer von mehreren Sensoren die Stellung von mehreren Klemmbacken erfasst.

Der Sensor 9 der in den Figuren dargestellten Ausführungsform eines erfindungsgemäßen Klemmhalters ist ein Mikroschalter. Der Mikroschalter 9 weist einen beweglichen Stift 10 auf, der von einer an der beweglichen Klemmbacke 3 angeordneten Erhebung 11 entgegen der Wirkung einer Feder eingedrückt wird, wenn sich die bewegliche Klemmbacke 3 in der geschlossenen Stellung der Klemmbacken befindet. Durch das Eindrücken des Stiftes 10 wird ein Stromkreis geschlossen und der Sensor 9 erfasst die geschlossene Stellung der Klemmbacken.

Mit einem erfindungsgemäßen Klemmhalter kann somit kostengünstig und mit einfacher Bedienung die ordnungsgemäße Bestückung einer medizinischen Behandlungsvorrichtung mit dem richtigen medizinischen Einmalartikel überwacht werden.

## Patentansprüche

1. Manuell zu öffnender Klemmhalter (1) für die lösbare Befestigung eines Einmalartikels an einer medizinischen Behandlungsvorrichtung, wobei der Klemmhalter (1) mindestens zwei zusammenwirkende Klemmbacken (2, 3) aufweist, von denen zumindest eine Klemmbacke (3) relativ zu der anderen Klemmbacke (2) um eine Drehachse (5) bewegbar ist und von denen mindestens eine Klemmbacke (3) gegen die Wirkung eines Schließelements (7) von einer geschlossenen Stellung der Klemmbacken (2, 3) in eine geöffnete Stellung der Klemmbacken (2, 3) manuell zu bewegen ist, **dadurch gekennzeichnet, dass** der Klemmhalter (1) einen Sensor (9) zur Erfassung der Stellung der Klemmbacken (2, 3) aufweist, wobei der Sensor (9) einen Winkel (α), der von zwei Klemmbacken (2, 3) gebildet wird, erfasst.

2. Klemmhalter nach Anspruch 1, wobei der Sensor (9) erfasst, ob sich die Klemmbacken (2, 3) in der geschlossenen Stellung der Klemmbacken (2, 3) oder nicht in der geschlossenen Stellung der Klemmbacken (2, 3) befinden.

3. Klemmhalter nach Anspruch 1, wobei der Sensor (9) die Stellung mindestens eines Klemmbackens (3) in einem Bereich zwischen der geschlossenen Stellung der Klemmbacken (2, 3) und einer vollständig geöffneten Stellung der Klemmbacken (2, 3) erfasst.

4. Klemmhalter nach einem der vorherigen Ansprüche, wobei der Sensor (9) ein Hall-Sensor ist.

5. Klemmhalter nach einem der vorherigen Ansprüche, wobei das Schließelement eine Schraubendrehfeder (7) aufweist.

6. Klemmhalter nach einem der vorherigen Ansprüche, wobei der Einmalartikel ein Adsorber, ein Dialysefilter oder eine Infusionsflasche ist.

7. Dialysegerät aufweisend einen Klemmhalter (1) nach einem der vorherigen Ansprüche.

8. System aufweisend einen Klemmhalter (1) nach einem der Ansprüche 1 bis 6 und eine Auswerteeinheit zur Auswertung der vom Sensor (9) erfassten Stellung der Klemmbacken (2, 3) sowie zur Feststellung einer Abweichung der erfassten Stellung der Klemmbacken (2, 3) von einer vorgegebenen Stellung der Klemmbacken (2, 3).

9. System nach Anspruch 8, wobei das System ferner Mittel für eine optische Signalisierung einer festgestellten Abweichung der erfassten Stellung der Klemmbacken (2, 3) von einer vorgegebenen Stellung der Klemmbacken (2, 3) aufweist.

10. System nach Anspruch 8, wobei das System ferner Mittel für eine akustische Signalisierung einer festgestellten Abweichung der erfassten Stellung der Klemmbacken (2, 3) von einer vorgegebenen Stellung der Klemmbacken (2, 3) aufweist.

11. System nach Anspruch 8, wobei das System ferner Mittel für eine haptische Signalisierung einer festgestellten Abweichung der erfassten Stellung der Klemmbacken (2, 3) von einer vorgegebenen Stellung der Klemmbacken (2, 3) aufweist.

12. Medizinisches Blutbehandlungsgerät aufweisend ein System nach einem der Ansprüche 8 bis 11.

13. Medizinisches Blutbehandlungsgerät nach Anspruch 12, wobei das medizinische Blutbehandlungsgerät ein Gerät zur Adsorptionstherapie oder ein Gerät zur Infusion medizinischer Flüssigkeiten oder ein Gerät zur Dialyse oder ein Gerät zur Plasmapherese ist.

14. Verfahren zur Bestückung einer medizinischen Behandlungsvorrichtung mit einem Einmalartikel zur Verwendung mit der medizinischen Behandlungsvorrichtung, wobei die medizinische Behandlungsvorrichtung einen manuell zu öffnenden Klemmhalter (1) aufweist, der mindestens zwei zusammenwirkende Klemmbacken (2, 3), von denen zumindest eine Klemmbacke (3) relativ zu der anderen Klemmbacke (2) um eine Drehachse (5) bewegbar ist und von denen mindestens eine Klemmbacke (3) gegen die Wirkung eines Schließelements (7) von einer geschlossenen Stellung der Klemmbacken (2, 3) in eine geöffnete Stellung der Klemmbacken (2, 3) manuell zu bewegen ist, und einen Sensor (9) zur Erfassung der Stellung der Klemmbacken (2, 3) aufweist, wobei das Verfahren die Schritte aufweist:
manuelles Aufhalten der Klemmbacken (2, 3) des Klemmhalters (1),
Einführen des Einmalartikels zwischen die Klemmbacken (2, 3),
Loslassen der Klemmbacken (2, 3),
Schließen der Klemmbacken (2, 3) durch die Wirkung des Schließelements (7) bis zum Kontakt der Klemmbacken (2, 3) mit dem Einmalartikel,
Erfassen der Stellung der Klemmbacken (2, 3) um den Einmalartikel mittels des Sensors (9),
Übermitteln vom Sensor (9) erfasster Daten bezüglich der Stellung der Klemmbacken (2, 3) an eine Auswerteeinheit,
Vergleichen in der Auswerteeinheit der Daten der erfassten Stellung der Klemmbacken (2, 3) mit Daten für eine vorgegebene Stellung der Klemmbacken (2, 3) und Signalisieren einer Warnung beim Vorliegen einer Abweichung der Daten der erfassten Stellung der Klemmbacken (2, 3) von den Daten der vorgegebenen Stellung der Klemmbacken (2, 3).

15. Verfahren nach Anspruch 14, ferner aufweisend den Schritt des Blockierens und/oder Unterbrechens der Ausführung von Funktionen auf der medizinischen Behandlungsvorrichtung beim Vorliegen einer Abweichung der Daten der erfassten Stellung der Klemmbacken (2, 3) von den Daten der vorgegebenen Stellung der Klemmbacken (2, 3).

## Claims

1. Manually openable clamping holder (1) for the releasable fixing of a single-use article to a medical treatment apparatus, wherein the clamping holder (1) comprises at least two cooperating clamping jaws (2, 3), of which at least one clamping jaw (3) is displaceable relative to the other clamping jaw (2) about a rotation axis (5) and of which at least one clamping jaw (3) can be moved manually against the action of a closing element (7) from a closed position of the clamping jaws (2, 3) to an open position of the clamping jaws (2, 3), **characterised in that** the clamping holder (1) comprises a sensor (9) for detecting the position of the clamping jaws (2, 3), wherein the sensor (9) detects an angle (α) that is formed by two clamping jaws (2, 3).

2. Clamping holder according to claim 1, wherein the sensor (9) detects whether the clamping jaws (2, 3) are in the closed position of the clamping jaw (2, 3) or are not in the closed position of the clamping jaws (2, 3).

3. Clamping holder according to claim 1, wherein the sensor (9) detects the position of at least one clamping jaw (3) in a region between the closed position of the clamping jaws (2, 3) and a fully open position of the clamping jaws (2, 3).

4. Clamping holder according to one of the preceding claims, wherein the sensor (9) is a Hall effect sensor.

5. Clamping holder according to one of the preceding claims, wherein the closing element comprises a helical torsion (7) spring.

6. Clamping holder according to one of the preceding claims, wherein the single-use device is an adsorber, a dialysis filter or an infusion bottle.

7. Dialysis apparatus comprising a clamping holder (1) according to one of the preceding claims.

8. System comprising a clamping holder (1) according to one of claims 1 to 6 and an evaluation unit for evaluating the position of the clamping jaw (2, 3) detected by the sensor (9) and also for detecting a deviation of the detected position of the clamping jaws (2, 3) from a predetermined position of the clamping jaws (2, 3).

9. System according to claim 8, wherein the system furthermore comprises means for an optical signalling of a detected deviation of the detected position of the clamping jaws (2, 3) from a predetermined position of the clamping jaws (2, 3).

10. System according to claim 8, wherein the system furthermore comprises means for an acoustic signalling of a detected deviation of the detected position of the clamping jaws (2, 3) from a predetermined position of the clamping jaws (2, 3).

11. System according to claim 8, wherein the system furthermore comprises means for a haptic signalling of a detected deviation of the detected position of the clamping jaws (2, 3) from a predetermined position of the clamping jaws (2, 3).

12. Medical blood treatment apparatus comprising a system according to one of clams 8 to 11.

13. Medical blood treatment apparatus according to claim 12, wherein the medical blood treatment device is an apparatus for adsorption therapy or an apparatus for the infusion of medical fluids or an apparatus for dialysis or an apparatus for plasmapheresis.

14. Method for equipping a medical treatment apparatus with a single-use article for use with the medical treatment apparatus, wherein the medical treatment apparatus comprises a manually openable clamping holder (1), which has at least two cooperating clamping jaws (2, 3), of which at least one clamping jaw (3) is moveable relative to the other clamping jaw (2) about a rotation axis (5) and of which at least one clamping jaw (3) is manually moveable against the action of a closing element (7) from a closed position of the clamping jaws (2, 3) to an open position of the clamping jaws (2, 3), and a sensor (9) for detecting the position of the clamping jaws (2, 3), wherein the method comprises the steps of:
manually holding the clamping jaws (2, 3) of the clamping holder (1) open,
inserting the single-use article between the clamping jaws (2 3),
releasing the clamping jaws (2, 3),
closing the clamping jaws (2, 3) by the action of the closing element (7) until the clamping jaws (2, 3) contact the single-use article,
detection of the position of the clamping jaws (2, 3) around the single-use article by means of the sensor (9),
transmitting data collected by the sensor (9) relating to the position of the clamping jaws (2, 3) to an evaluation unit,
comparing in the evaluation unit the data of the detected position of the clamping jaws (2, 3) with data for a predetermined position of the clamping jaws (2, 3), and signalling a warning if there is a deviation of the data of the detected position of the clamping jaws (2, 3) from the data of the predetermined position of the clamping jaws (2, 3).

15. Method according to claim 14, furthermore comprising the step of blocking and/or interrupting the execution of functions on the medical treatment apparatus if there is a deviation of the data of the detected position of the clamping jaws (2, 3) from the data of the predetermined position of the clamping jaws (2, 3).

## Revendications

1. Pince (1) à ouverture manuelle pour la fixation amovible d'un article à usage unique sur un dispositif médical de traitement, la pince (1) comportant au moins deux mâchoires de serrage (2, 3) qui coopèrent, dont au moins une mâchoire (3) est mobile par rapport à l'autre mâchoire de serrage (2) autour d'un axe de rotation (5) et dont au moins une mâchoire (3) doit être déplacée manuellement d'une position fermée des mâchoires de serrage (2, 3) à une position ouverte des mâchoires de serrage (2, 3) contre l'action d'un élément de fermeture (7), **caractérisée en ce que** la pince (1) comporte un capteur (9) pour détecter la position des mâchoires de serrage (2, 3), le capteur (9) détectant un angle (α) qui est formé par deux mâchoires de serrage (2, 3).

2. Pince selon la revendication 1, dans laquelle le capteur (9) détecte si les mâchoires de serrage (2, 3) sont dans la position fermée des mâchoires de serrage (2, 3) ou ne sont pas dans la position fermée des mâchoires de serrage (2, 3).

3. Pince selon la revendication 1, dans laquelle le capteur (9) détecte la position d'au moins une mâchoire de serrage (3) dans une zone entre la position fermée des mâchoires de serrage (2, 3) et une position complètement ouverte des mâchoires de serrage (2, 3).

4. Pince selon l'une des revendications précédentes, dans laquelle le capteur (9) est un capteur à effet Hall.

5. Pince selon l'une des revendications précédentes, dans laquelle l'élément de fermeture comporte un ressort cylindrique de torsion (7).

6. Pince selon l'une des revendications précédentes, dans laquelle l'article à usage unique est un adsorbeur, un filtre de dialyse ou un flacon de soluté.

7. Appareil de dialyse comportant une pince (1) selon l'une quelconque des revendications précédentes.

8. Système comportant une pince (1) selon l'une quelconque des revendications 1 à 6 et une unité d'évaluation pour évaluer la position, détectée par le capteur (9), des mâchoires de serrage (2, 3) ainsi que pour constater un écart entre la position détectée des mâchoires de serrage (2, 3) et une position prescrite des mâchoires de serrage (2, 3).

9. Système selon la revendication 8, dans lequel le système comporte en outre des moyens pour signaler de façon visuelle un écart constaté entre la position détectée des mâchoires de serrage (2, 3) et une position prescrite des mâchoires de serrage (2, 3).

10. Système selon la revendication 8, dans lequel le système comporte en outre des moyens pour signaler de façon sonore un écart constaté entre la position détectée des mâchoires de serrage (2, 3) et une position prescrite des mâchoires de serrage (2, 3).

11. Système selon la revendication 8, dans lequel le système comporte en outre des moyens pour signaler de façon tactile un écart constaté entre la position détectée des mâchoires de serrage (2, 3) et une position prescrite des mâchoires de serrage (2, 3).

12. Appareil médical de traitement du sang comportant un système selon l'une quelconque des revendications 8 à 11.

13. Appareil médical de traitement du sang selon la revendication 12, dans lequel l'appareil médical de traitement du sang est un appareil pour la thérapie avec absorption ou un appareil pour la perfusion de liquides médicaux ou un appareil pour la dialyse ou un appareil pour la plasmaphérèse.

14. Procédé pour équiper un dispositif médical de traitement avec un article à usage unique à utiliser avec le dispositif médical de traitement, le dispositif médical de traitement comportant une pince (1) à ouverture manuelle qui comporte au moins deux mâchoires de serrage (2, 3) qui coopèrent, dont au moins une mâchoire (3) est mobile par rapport à l'autre mâchoire de serrage (2) autour d'un axe de rotation (5) et dont au moins une mâchoire (3) doit être déplacée manuellement d'une position fermée des mâchoires de serrage (2, 3) à une position ouverte des mâchoires de serrage (2, 3) contre l'action d'un élément de fermeture (7), et un capteur (9) pour détecter la position des mâchoires de serrage (2, 3), le procédé comportant les étapes suivantes :
- maintien manuel des mâchoires de serrage (2, 3) de la pince (1) ouvert,
- introduction de l'article à usage unique entre les mâchoires de serrage (2, 3),
- relâchement des mâchoires de serrage (2, 3),
- fermeture des mâchoires de serrage (2, 3) par l'action de l'élément de fermeture (7) jusqu'au contact des mâchoires de serrage (2, 3) avec l'article à usage unique,
- détection de la position des mâchoires de serrage (2, 3) autour de l'article à usage unique au moyen du capteur (9),
- transmission de données détectées par le capteur (9) et relatives à la position des mâchoires de serrage (2, 3) à une unité d'évaluation,
- comparaison, dans l'unité d'évaluation, les données de la position détectée des mâchoires de serrage (2, 3) à des données pour une position prescrite des mâchoires de serrage (2, 3) et
- signalisation d'un avertissement en présence d'un écart entre les données de la position détectée des mâchoires de serrage (2, 3) et les données de la position prescrite des mâchoires de serrage (2, 3).

15. Procédé selon la revendication 14, comportant en outre le blocage et/ou l'interruption de l'exécution de fonctions sur le dispositif médical de traitement en présence d'un écart entre les données de la position détectée des mâchoires de serrage (2, 3) et les données de la position prescrite des mâchoires de serrage (2, 3).
